# EUROPÄISCHE PATENTSCHRIFT

(11) **EP 0 583 456 B1**
(45) Veröffentlichungstag und Bekanntmachung des Hinweises auf die Patenterteilung: **25.09.1996**
(21) Anmeldenummer: 93905073.8
(22) Anmeldetag: 08.03.1993
(51) Int. Cl.: A61B 5/22, A63B 23/20

(54) **EINRICHTUNG ZUR ÜBERWACHUNG DER KONTRAKTIONSFÄHIGKEIT DER BECKENBODENMUSKULATUR**
DEVICE FOR MONITORING THE CONTRACTIBILITY OF THE PELVIC FLOOR MUSCLES
DISPOSITIF DE CONTROLE DE LA CONTRACTILITE DE LA MUSCULATURE DU PLANCHER PELVIEN

(30) Priorität: 09.03.1992 AT 454/92
(43) Veröffentlichungstag der Anmeldung: 23.02.1994
(73) Patentinhaber: Pauser, Alexander, Mag. Dr., A-1170 Wien (AT); Michl, Ilse, Dr., A-1160 Wien (AT)
(72) Erfinder: Pauser, Alexander, Mag. Dr., A-1170 Wien (AT); Michl, Ilse, Dr., A-1160 Wien (AT)
(74) Vertreter: Miksovsky, Alexander, Dipl.-Ing.
(86) Internationale Anmeldenummer: AT9300041
(87) Internationale Veröffentlichungsnummer: WO9317619

(56) Entgegenhaltungen:
- WO-A-92/20283
- CH-A- 346 971
- GB-A- 2 137 499
- US-A- 3 726 273
- US-A- 4 050 449
- US-A- 4 873 990

## Beschreibung

Die Erfindung bezieht sich auf eine Einrichtung zur Überwachung der Kontraktionsfähigkeit der Beckenbodenmuskulatur, wobei ein Sondenkopf über wenigstens eine Meßleitung mit einem Anzeigegerät verbindbar ist und der Sondenkopf als langgestreckter Körper ausgebildet ist, welcher in seinem mittleren Bereich eine Einschnurung trägt, und wobei der Sondenkopf an seinem Umfang wenigstens einen Drucksensor bzw. voneinander getrennte, elastisch gegen ein Fluid verformbare Bereiche aufweist.

Eine Einrichtung zur Überwachung der Kontraktionsfähigkeit der Beckenbodenmuskulatur ist beispielsweise aus der DD-PS 157 594 bekanntgeworden, wobei ein an einer Seite verschlossenes Röhrchen teilweise von einem flexiblen Mantel umgeben ist und über eine entsprechende Meßeinrichtung eine Einwirkung durch den zu überwachenden Schließmuskel auf den Sondenkopf feststellbar ist. Eine ähnliche Ausbildung ist der CH-PS 346 971 zu entnehmen, wobei ein zylindrischer Hohlkörper mittels eines mit einem Manometer verbundenen Gebläses aufblasbar ist. Weiters ist der WO-A-92/20283 (Priorität 20.5.1991) eine ähnliche Ausbildung zu entnehmen, bei welcher ein länglicher Sondenkopf, welcher eine flexible Ummamntelung aufweist und mit einer Druckmittelquelle verbindbar ist, eine Anzeige eines auf den Sondenkopf durch die Beckenbodenmuskulatur ausgeübten Druckes ermöglicht. Nachteilig bei diesen Ausbildungen ist die Tatsache, daß jegliche über die gesamte Länge auf den Sondenkopf einwirkende Beanspruchung eine Anzeige nach sich zieht, ohne daß genau abgegrenzt werden könnte, daß die Anzeige durch Betätigung eines bestimmten Muskelbereiches hervorgerufen wurde.

Eine ähnliche Ausführungsform ist auch der US-PS 4 768 522 zu entnehmen, wobei in den Sondenkopf ein Federelement eingebettet ist, um eine exakte Positionierung zu ermöglichen.

Aus der DE-OS 32 21 115 ist eine Vorrichtung zur Messung der Kontraktionsfähigkeit von Ringmuskeln bekanntgeworden, wobei eine in eine Körperöffnung einführbare Sonde einen Innenkörper aufweist, welcher über einen Teil seiner Länge von einem Schlauch aus einem dünnen elastisch dehnbaren Material umhüllt ist, wobei der Schlauch über wenigstens eine Öffnung des Innenkörpers mit dessen Innenraum in Verbindung steht. Die Meßleitung ist bei dieser bekannten Ausführungsform als konzentrisches Doppelrohr ausgebildet, wobei insbesondere darauf abgezielt wird, die Sonde beispielsweise zur Messung von Darmkontraktionen zu verwenden, so daß eine ausreichende Flexibilität des Sondenkopfes über einen weiten Bereich notwendig ist.

Aus der US-A 3 726 273 ist eine Einrichtung der eingangs genannten Art bekannt geworden, wobei ein Sondenkopf eine Mehrzahl von flexiblen, ballonartigen Elementen aufweist, welche mit Preßluft aufblasbar sind, wobei zwischen den einzelnen ballonartigen Elementen im wesentlichen starre Verbindungsstangen vorgesehen sind. Die ballonartigen Elemente sind darüberhinaus mit entsprechenden Druckluftleitungen und Druckmeßgeräten verbunden.

Die Beckenbodenmuskulatur erfüllt im weiblichen Körper unter anderem die Aufgabe, Harn zurückzuhalten und die Harnleiter zuverlässig abzusperren. Die Beckenbodenmuskulatur ist hiebei in einer Muskelplatte angeordnet und weist im wesentlichen einen zentralen Ringmuskel auf. Die Beckenbodenmuskulatur wird bei Frauen in der Schwangerschaft relativ hoch beansprucht, wodurch die Muskeln ein gewisses Maß an Festigkeit und Kraft entwickeln. Bei der Geburt wird die Beckenbodenmuskulatur jedoch in einem sehr hohen Ausmaß gedehnt und auch eine durch die Schwangerschaft in gewissem Maß trainierte Muskulatur kann nach einer derartigen gewaltsamen Dehnung bzw. Überdehnung nicht sofort die gewünschte Funktion wiederum aufnehmen. In der Folge ist somit insbesondere bei mehrfach Gebärenden die Gefahr einer Inkontinenz zumindest im Zeitraum nach der Geburt besonders hoch und es werden aus diesem Grund nach der Schwangerschaft eine Reihe von Trainingsprogrammen für überbeanspruchte bzw. ihre Funktion nicht mehr vollständig gewährleistende Muskeln in Spitälern angeboten.

Bei der häufigsten Indikation, nämlich Inkontinenz, muß die Patientin durch gezielte Übungen die willkürlich betätigbare Beckenmuskulatur anspannen, wobei gleichzeitig darauf geachtet werden muß, daß tatsächlich diejenige Muskulatur, welche zu trainieren ist, angespannt wird, und nicht gleichzeitig eine Preßbeanspruchung anderer Muskel ausgelöst wird. Auch eine Betätigung anderer Muskel kann dem Therapeuten und der Patientin mit bekannten Einrichtungen den Eindruck vortäuschen, daß ein korrektes Muskeltraining erfolgt, und es ist bei derartigen Übungen bisher nicht ohne weiteres möglich, den Therapieerfolg in irgendeiner Weise zu überwachen.

Die Erfindung zielt nun darauf ab, eine Einrichtung der eingangs genannten Art zu schaffen, mit welcher ein therapeutisches Programm und insbesondere der Trainingserfolg in einfacher Weise überwacht und angezeigt werden kann. Zur Lösung dieser Aufgabe ist die erfindungsgemäße Einrichtung im wesentlichen dadurch gekennzeichnet, daß der mittlere eingeschnürte Bereich des Sondenkopfes einen elastisch verformbaren Mantel aufweist, dessen Verformbarkeit größer ist als die Verformbarkeit der an den mittleren Bereich anschließenden Teilbereiche des Sondenkopfes. Durch entsprechende Positionierung wenigstens eines Drucksensors am Umfang bzw. nahe dem Umfang des Sondenkopfes gelingt es, gezielt die Betätigung spezieller Muskelpartien zu überwachen. Eine ähnliche spezielle Überwachung einzelner Muskelpartien läßt sich auch durch eine Anordnung einer Mehrzahl voneinander getrennter elastischer und durch ein Fluid verformbarer Bereich erzielen. Erfindungsgemäß wird somit sichergestellt, daß die Einrichtung nach Verankerung an einer für die Messung bzw. Anzeige geeigneten Position tatsächlich lediglich Meßwerte im Bereich der zu trainierenden Muskulatur ergibt. Mit der erfindungsgemäßen Ausbildung wird somit selektiv die Kontraktionsfähigkeit der ringmuskelartigen Beckenbodenmuskulatur gemessen, wobei störende Einflüsse, wie sie durch Pressen, insbesondere Bauchpressen, entstehen würden, weitestgehend von der Meßstelle ferngehalten werden. Um derartiges Bauchpressen auch über den Umweg einer axialen Kompression nicht als Meßwertverfälschung zur Wirkung kommen zu lassen, ist darüberhinaus mit Vorteil die Ausbildung so getroffen, daß der mittlere Bereich in Achsrichtung des Sondenkopfes verlaufende Versteifungen, insbesondere eine die den eingeschnürten Bereich in Achsrichtung durchsetzende, mit den dem mittleren Bereich benachbarten, weniger verformbaren Bereichen verbundene Stange aufweist.

Neben der Verwendung von Druckmeßsensoren beispielsweise in Form von Dehnungsmeßstreifen, wie dies einer bevorzugten Ausführungsform entspricht, welche über eine elektrische Meßleitung mit einem entsprechenden, eine Auswerteschaltung und ein Anzeigeinstrument enthaltenden Meßgerät verbunden sind, ist vor allen Dingen eine besonders einfache Einrichtung dadurch erzielbar, daß elastisch gegen ein Fluid verformbare Bereiche am Sondenkopf vorgesehen sind, wobei vorzugsweise die Meßleitung als Fluidleitung ausgebildet ist und mit einer Druckmeßdose sowie einem mit der Druckmeßdose verbundenen Anzeigegerät verbindbar ist. Bei einer derartigen Ausbildung können die im medizinisch-technischen Bereich üblichen Meßgeräte, wie sie beispielsweise für die Blutdruckmessung Verwendung finden, ohne nennenswerte Modifikationen unmittelbar Verwendung finden, wobei allerdings bevorzugt ein flüssiges Fluid und insbesondere als Fluid physiologisch unbedenkliche Flüssigkeiten, insbesondere aqua bidestillata, eingesetzt sind.

In besonders einfacher Weise kann mit der erfindungsgemäßen Einrichtung zusätzlich auch der Effekt des unerwünschten Bauchpressens gesondert angezeigt werden. Um auf diese Weise den Therapieerfolg dahingehend zu verbessern, daß der Patientin noch deutlicher zum Bewußtsein kommt, welches die zu trainierende Muskulatur ist, ist die Ausbildung mit Vorteil so getroffen, daß der Sondenkopf an seinem freien Ende wenigstens einen gesonderten Drucksensor bzw. eine von einer elastisch verformbaren Membran begrenzte Kammer aufweist, wobei eine gesonderte Meßleitung mit einem Meß- und Anzeigegerät verbindbar ist. Ein derartiger zweiter, gesonderter Drucksensor, welcher über eine gesonderte Leitung herausgeführt ist, ermöglicht es, zusätzliche Meßwerte zur Verfügung zu stellen, wel-che unmittelbar erkennen lassen, daß ein Anstieg der Meßwerte dieses zusätzlichen Drucksensors nicht dem gewünschten Therapieerfolg dienen kann.

Um den Trainingseffekt zu erhöhen und eine Anpaßbarkeit an unterschiedliche Ausgangsbedingungen zu ermöglichen, ist die Ausbildung mit Vorzug so getroffen, daß an die verformbaren Bereiche bzw. an die Meßleitung unter Zwischenschaltung einer Ventilanordnung, insbesondere eines Rückschlagventils, eine Druckquelle anschließbar ist. Damit gelingt es, in den verformbaren Bereichen ein Ausgangsdruckniveau in weiten Grenzen beliebig vorzugeben und bei fortschreitendem Training der entsprechenden Muskulatur durch Erhöhung des Grunddruckes die Trainingsleistung zu steigern. Für eine einfache Handhabung kann dabei das Meßgerät unabhängig vom gewählten Ausgangsdruck jeweils eine rücksetzbare Nullanzeige aufweisen. Bei Verwendung eines Fluids als Medium für die Druckanzeige wird in einfacher Weise die Druckquelle mit gleichem Fluid betrieben und dient gleichzeitig als Fluidreservoir. Bei Einsatz von beispielsweise Drucksensoren in Form von Dehnungsmeßstreifen am Umfang der verformbaren Bereiche mündet die Druckquelle über eine getrennte Leitung in die verformbaren Bereiche und kann beispielsweise als Druckluftquelle ausgebildet sein.

Bei Verwendung einer Mehrzahl voneinander getrennter elastisch gegen ein Fluid verformbarer Bereiche ist gemäß einer bevorzugten Ausführungsform die Ausbildung so getroffen, daß in radialer Richtung voneinander getrennte Kammern angeordnet sind, welche mit unterschiedlichem Druck mit Fluid beaufschlagbar sind. Dadurch gelingt es, dem von außen einwirkenden Druck einen beispielsweise nicht linear ansteigenden Gegendruck entgegenzusetzen, wodurch der Trainingseffekt verbessert und gesteigert werden kann.

Bei Anordnung eines Drucksensors im Bereich des Umfanges des Sondenkopfes ist gemäß einer bevorzugten Ausführungsform die Ausbildung so getroffen, daß der Sondenkopf wenigstens über einen Teil seiner Länge von einem Hohlzylinder gebildet ist, welcher einen in Längsrichtung verlaufenden Spalt aufweist, und daß am Innenumfang des Zylinders wenigstens ein mit der Meßleitung verbundener Dehnungsmeßstreifen angeordnet ist.

Eine derartige Ausbildung zeichnet sich durch eine besonders einfache Herstellung des den eigentlichen Meßbereich bzw. sensiblen Bereich des Sondenkopfes darstellenden Hohlzylinders aus und es können durch Verwendung wenigstens eines Dehnmeßstreifens bereits sehr geringe Druckunterschiede festgestellt werden, so daß geringste Verformungen des Zylinders bereits zu einem Meßergebnis führen. Der Zylinder kann dadurch relativ steif ausgebildet sein, so daß die eigentliche Form des Sondenkopfes nahezu unverändert bleibt. Hiebei kann vorgesehen sein, durch unterschiedlich dimensionierte Zylinder eine entsprechende Anpassung an anatomische Gegebenenheiten vorzunehmen. Um bei geringsten Druckbeanspruchungen das Meßergebnis zu optimieren und somit mit relativ einfachen Verstärkungs- und Auswerteschaltungen das Auslangen zu finden, ist die Ausbildung bevorzugt so getroffen, daß der Dehnungsmeßstreifen dem Spalt gegenüberliegend angeordnet ist.

Anstelle der Verwendung von Dehnungsmeßstreifen kann gemäß einer abgewandelten Ausführungsform die Ausbildung so getroffen sein, daß der Sondenkopf wenigstens über einen Teil seiner Länge von einem Hohlzylinder gebildet ist, welcher einen in Längsrichtung verlaufenden Spalt aufweist, und daß im Bereich des Spaltes mit der Meßleitung verbundene, elektrisch leitfähige Platten angeordnet sind, wodurch sich wiederum bei geringen Verformungen des den sensiblen Bereich des Sondenkopfes darstellen Hohlzylinders mit einfachen Mitteln reproduzierbare und leicht auswertbare Meßergebnisse erzielen lassen.

Um die Kompressibilität des sensiblen Bereiches des Sondenkopfes zu vergrößern, kann die Ausbildung bevorzugt so getroffen sein, daß der Sondenkopf mit einem elastischen Material, beispielsweise Schaumstoff, Kautschuk oder dgl., ummantelt ist.

Ein sehr einfacher Aufbau des Sondenkopfes und insbesondere des sensiblen Bereiches läßt sich gemäß einer weiteren bevorzugten Ausführungsform dadurch erzielen, daß der Sondenkopf elastische Bereiche aufweist, in welchen elektrisch leitfähiges Material, beispielsweise Kohlenstoffstaub, elektrisch leitende Flüssigkeiten oder Lösungen oder dgl., enthalten ist, und daß in dem elektrisch leitfähigen Material mit der Meßleitung verbundene Elektroden eingebettet sind. Durch entsprechende Wahl des elastischen Materials gelingt auch eine einfache Abstimmung der Kompressibilität der Vorrichtung.

Für eine leichte und einfache Handhabung und Positionierung des Sondenkopfes relativ zu den zu überwachenden Muskelpartien ist die Ausbildung bevorzugt so getroffen, daß an dem dem freien Ende des Sondenkopfes gegenüberliegenden Ende ein Positionieranschlag am Sondenkopf angeordnet ist, wobei für einen exakten Sitz die Ausbildung bevorzugt so getroffen ist, daß der Positionieranschlag von einer insbesondere flexiblen Kunststoffplatte gebildet ist, deren Außendurchmesser den Durchmesser des Sondenkopfes um wenigstens ein Drittel dieses Durchmessers übersteigt.

Die Handhabung der erfindungsgemäßen Einrichtung wird bevorzugt bei derartigen Ausbildungen dadurch so erleichtert, daß am Positionieranschlag ein Griff vorgesehen ist, wobei für eine Anpassung an teilweise unterschiedliche anatomische Merkmale die Ausbildung bevorzugt so getroffen ist, daß der Positionieranschlag in variablem Abstand vom freien Ende des Sondenkopfes an diesem festlegbar ist.

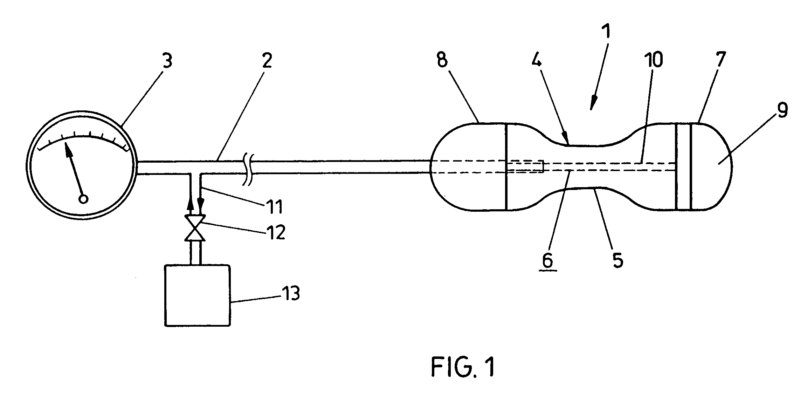

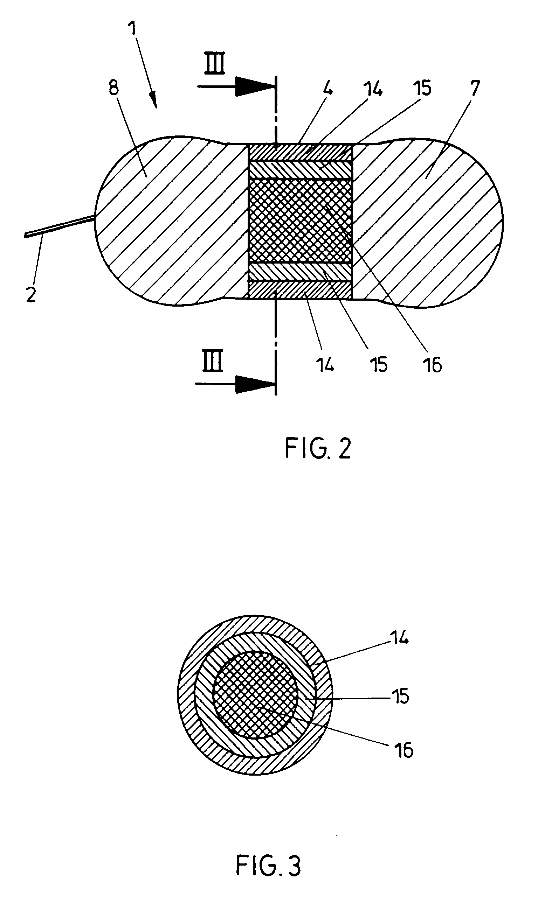

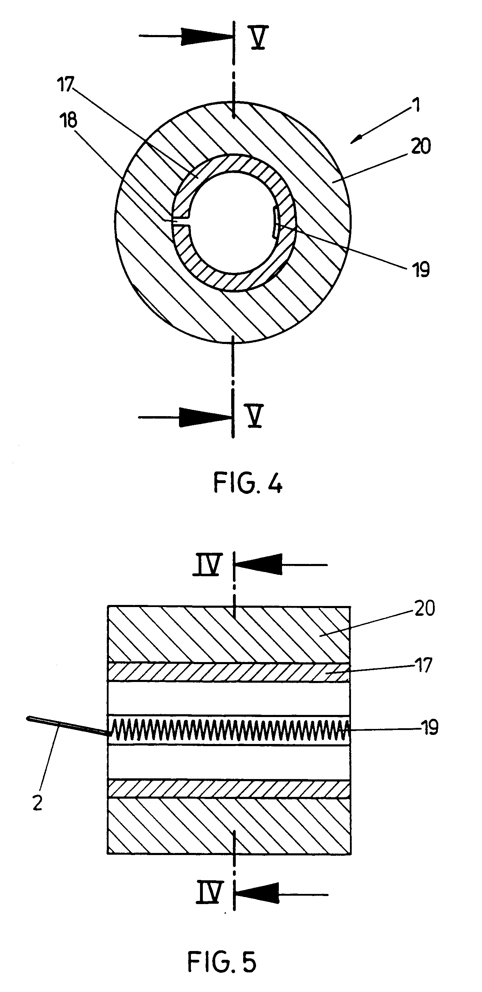

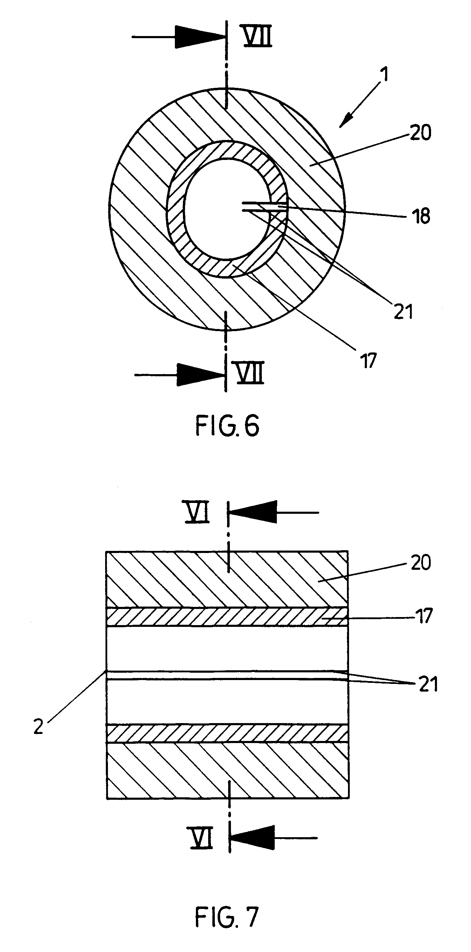

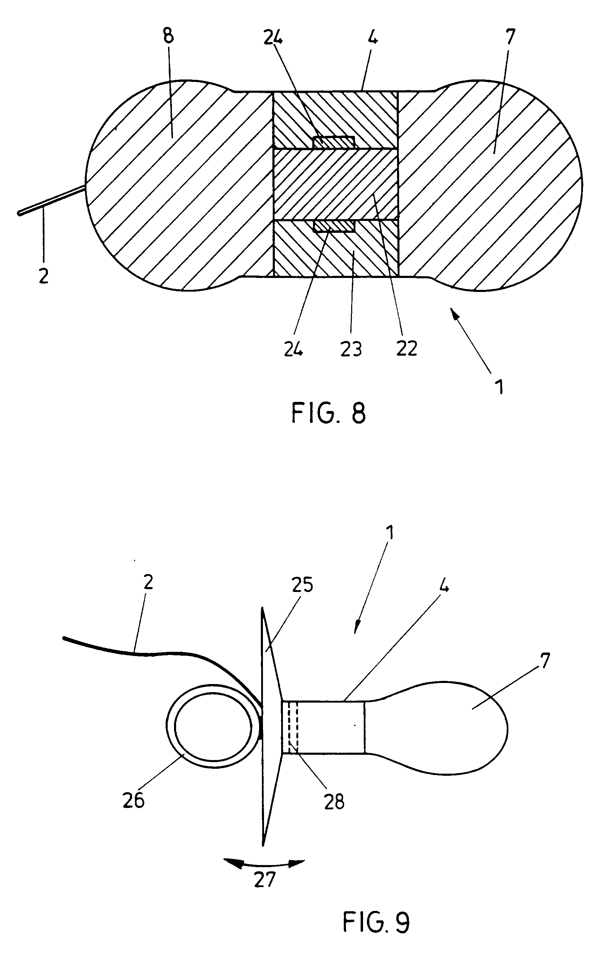

Die Erfindung wird nachfolgend an Hand von in der Zeichnung schematisch dargestellten Ausführungsbeispielen näher erläutert. In dieser zeigen: Fig.1 teilweise im Schnitt eine Ansicht einer ersten erfindungsgemäßen Einrichtung; Fig.2 teilweise im Schnitt eine Ansicht einer abgewandelten Ausführungsform eines Sondenkopfes einer erfindungsgemäßen Einrichtung; Fig.3 einen Schnitt nach der Linie III-III der Fig.2; Fig.4 in einer zu Fig.3 ähnlichen Darstellung einen Schnitt durch eine abgewandelte Ausführungsform einer erfindungsgemäßen Einrichtung unter Verwendung eines Dehnungsmeßstreifens; Fig.5 einen Schnitt nach der Linie V-V der Fig.4, wobei Fig.4 einen Schnitt nach der Linie IV-IV der Fig.5 darstellt; Fig.6 in einer zu Fig.4 analogen Darstellung einen Schnitt durch eine abgewandelte Ausführungsform unter Verwendung von von elektrisch leitfähigen Platten als Drucksensoren; Fig.7 einen Schnitt nach der Linie VII-VII der Fig.6, wobei Fig.6 einen Schnitt nach der Linie VI-VI der Fig.7 darstellt; Fig.8 in einer Fig.2 ähnlichen Darstellung eine Ansicht teilweise im Schnitt durch eine weitere abgewandelte Ausführungsform eines Sondenkopfes einer erfindungsgemäßen Einrichtung; und Fig.9 eine schematische Ansicht eines weiteren abgewandelten Sondenkopfes einer erfindungsgemäßen Einrichtung mit einem insbesondere in unterschiedlichen Positionen festlegbaren Positionieranschlag am Sondenkopf.

In Fig.1 ist ein in die Vagina einzuführender Sondenkopf mit 1 bezeichnet, welcher über eine Meßleitung 2 mit einem Meß- und Anzeigegerät 3 verbunden ist. Der Sondenkopf 1 weist einen mittleren eingeschnürten Bereich 4 auf, welcher von einer elastisch verformbaren Membran 5 ummantelt ist. In die von diesem elastisch verformbaren Mantel 5 begrenzte Kammer 6 taucht eine Fluidleitung ein, welche mit einer Barometermeßdose und einem Anzeigegerät 3 verbunden ist. An den mittleren eingeschnürten Bereich 4 schließen vergleichsweise steifere und weniger kompressible Bereiche 7 und 8 an, wobei insbesondere im Endbereich 7 des Sondenkopfes 1 ein zusätzlicher Drucksensor 9 angeordnet sein kann, welcher über eine weitere, nicht näher dargestellte Fluid- bzw. Meßleitung mit einem weiteren Meßgerät verbunden ist. Alternativ kann das Meßgerät 2 zwei Skalen bzw. Anzeigen aufweisen oder umschaltbar sein.

Die beiden vergleichsweise geringer komprimierbaren Teilbereiche 7 und 8 des Sondenkopfes können über ein axiales Verbindungsstück bzw. eine Stange 10 miteinander verbunden sein, um eine Kompression und damit einen Druckanstieg in der Kammer 6 zu verhindern, wenn Druck auf den Endbereich 8 des Sondenkopfes ausgeübt wird.

Bei mit einem Fluid gefüllter Kammer kann weiters zur Einstellung eines Ausgangsdruckniveaus in unterschiedlicher Höhe an die Fluidleitung 2 über eine Leitung 11 mit einem Rückschlagventil 12 eine Druckquelle 13 für das Fluid angeschlossen werden. Diese Druckquelle 13 dient darüberhinaus als Fluidvorrat. Alternativ kann die Leitung 12 direkt in die von der Membran 5 begrenzte Kammer 6 geführt sein. Für eine erleichterte Handhabung weist das Meßgerät 2 in diesem Fall eine Reset-Taste für eine Rückstellung auf einen Nullwert auf, um unabhängig vom Ausgangsdruck eine Überwachung der Trainingsleistung zu ermöglichen.

In Fig.2 und 3 ist eine abgewandelte Ausführungsform dargestellt, wobei im mittleren Bereich 4, welcher den Meßbereich darstellt, eine Mehrzahl von in radialer Richtung voneinander getrennten Kammern 14, 15 und 16, vorgesehen ist, welche über die schematisch angedeutete Meß- bzw. Fluidleitung 2 mit einem Meßgerät und/oder einer Druckquelle analog der Ausführungsform gemäß Fig.1 verbunden sind. Durch die Anordnung von mehreren radial voneinander getrennten Kammern wird es möglich, dem von außen einwirkenden Druck beispielsweise einen nicht-linear ansteigenden Gegendruck entgegenzusetzen, wodurch der Trainingseffekt verbessert werden kann. So kann beispielsweise der Druck in den Kammern von außen nach innen ansteigen, so daß im wesentlichen erst nach Komprimierung des Fluids in einer außen liegenden Kammer die nächstfolgende weiter innen liegende Kammer beaufschlagt wird. Bei Vorsehen einer Druckquelle, welche mit den radialen Kammern 14, 15 und 16 verbunden ist, ist es beispielsweise möglich, einen entsprechenden nichtlinearen verlaufenden Gegendruck aufzubauen, welcher der Federkennlinie einer nicht-linearen Feder entspräche.

Bei den in den Fig.4 bis 7 dargestellten Ausführungsformen ist der Sondenkopf 1 zumindest über einen Teil seiner axialen Erstreckung von einem Hohlzylinder 17 gebildet, welcher einen in Längsrichtung verlaufenden Spalt 18 aufweist. Bei den Ausführungsformen gemäß den Fig.4 und 5 ist an der Innenfläche des Zylinders ein Dehnungsmeßstreifen 19 angeordnet, welcher über die schematisch wiederum mit 2 bezeichnete Meßleitung mit einen nicht näher dargestellten Auswerte- bzw. Anzeigeeinheit verbunden ist. Durch Verwendung eines Dehnungsmeßstreifens können bereits geringfügige Druckeinwirkungen auf den Hohlzylinder festgestellt werden, so daß dieser relativ starr ausgebildet sein kann. Für eine optimale Signalauswertung ist der Dehnungsmeßstreifen bei der gezeigten Ausführungsform dem Spalt 18 gegenüberliegend angeordnet. Auf Grund der bekannten Empfindlichkeit von Dehnungsmeßstreifen und zur Erhöhung der Akzeptanz bei der Benutzung ist der Hohlzylinder 17 von einem elastischen Material 20 ummantelt, wobei als elastisches Material beispielsweise Schaumstoff, Kautschuk od.dgl. Verwendung finden kann.

Die Ausführungsform gemäß den Fig.6 und 7 unterscheidet sich von der vorausgehenden Ausführungsform dadurch, daß im Bereich des Spaltes 18 elektrisch leitfähige Platten 21 vorgesehen sind, welche insgesamt einen Kondensator ausbilden. Durch Verformung des Zylinders bei Einwirken einer Druckbeanspruchung läßt sich von den einen Kondensator bildenden elektrisch leitfähigen Platten 21 eine Meßgröße gewinnen, welche unmittelbar eine Überwachung der Kontraktionsfähigkeit der Beckenbodenmuskulatur ermöglicht.

Bei der Ausbildung gemäß Fig.8 ist um einen zylindrischen Kern 22 ein elastisches, schwammartiges, elektrisch nicht-leitendes Material 23 angeordnet. In die Poren des Materials 23 wird ein Material eingelagert, welches eine gewisse elektrische Leitfähigkeit aufweist und beispielsweise von Kohlenstoffstaub, elektrisch leitenden Flüssigkeiten oder Lösungen od.dgl. gebildet ist. In das Material 23 sind Elektroden 24 eingebettet, welche mit der wiederum schematisch mit 2 angedeuteten Meßleitung verbunden sind. Durch Kompression des Materials 23 werden die Poren zusammengedrückt, wodurch sich die Kontaktfläche für das elektrisch leitende Material verändert, wodurch wieder ein entsprechendes Signal gewonnen werden kann.

Bei der Ausbildung gemäß Fig.9 kann der Meßbereich 4 des Sondenkopfes 1 gemäß einer der vorangehenden Ausführungsformen ausgebildet sein. Für eine verbesserte Handhabung und leichtere Applizierung sowie für die Einhaltung einer jeweils gleich bleibenden Position ist bei dieser Ausführungsform ein Positionieranschlag 25 vorgesehen, welcher beispielsweise von einer flexiblen Kunststoffplatte gebildet sein kann. Der Außendurchmesser des Positionieranschlages 25 überragt hiebei den Durchmesser des Sondenkopfes 1. Für ein Ergreifen des Sondenkopfes 1 ist an dem von einer Kunststoffplatte gebildeten Anschlag 25 ein Haltegriff 26 vorgesehen. Um eine Anpassung der Positionierung zu ermöglichen, ist der Positionieranschlag 25 im Sinne des Doppelpfeiles 27 relativ zum Meßbereich 4 des Sondenkopfes verschiebbar bzw. in unterschiedlichen Positionen festlegbar. Eine einfache Festlegung kann hiebei durch ein Verrasten in unterschiedlichen Positionen erfolgen, wobei mit 28 schematisch Nuten angedeutet sind, in welchen entsprechende Vorsprünge des Anschlages 25 einrastbar sind.

Weiters können Bimetall-Drucksensoren verwendet werden.

## Patentansprüche

1. Einrichtung zur Überwachung der Kontraktionsfähigkeit der Beckenbodenmuskulatur, wobei ein Sondenkopf (1) über wenigstens eine Meßleitung (2) mit einem Anzeigegerät (3) verbindbar ist und der Sondenkopf (1) als langgestreckter Körper ausgebildet ist, welcher in seinem mittleren Bereich (4) eine Einschnürung trägt, und wobei der Sondenkopf (1) an seinem Umfang wenigstens einen Drucksensor (19, 21, 24) bzw. voneinander getrennte, elastisch gegen ein Fluid verformbare Bereiche (6, 7, 14, 15, 16) aufweist, dadurch gekennzeichnet, daß der mittlere eingeschnürte Bereich (4) des Sondenkopfes (1) einen elastisch verformbaren Mantel aufweist, dessen Verformbarkeit größer ist als die Verformbarkeit der an den mittleren Bereich (4) anschließenden Teilbereiche (7,8) des Sondenkopfes.

2. Einrichtung nach Anspruch 1, dadurch gekennzeichnet, daß die Meßleitung (2) als Fluidleitung ausgebildet ist und mit einer Druckmeßdose (3) sowie einem mit der Druckmeßdose verbundenen Anzeigegerät verbindbar ist.

3. Einrichtung nach Anspruch 1 oder 2, dadurch gekennzeichnet, daß der mittlere Bereich (4) in Achsrichtung des Sondenkopfes (1) verlaufende Versteifungen (10), insbesondere eine die den eingeschnürten Bereich in Achsrichtung durchsetzende, mit den dem mittleren Bereich benachbarten weniger verformbaren Bereichen (7,8) verbundene Stange aufweist.

4. Einrichtung nach einem der Ansprüche 1 bis 3, dadurch gekennzeichnet, daß der Sondenkopf (1) an seinem freien Ende wenigstens einen gesonderten Drucksensor (9) bzw. eine von einer elastisch verformbaren Membran begrenzte Kammer aufweist, wobei eine gesonderte Meßleitung mit einem Meß- und Anzeigegerät verbindbar ist.

5. Einrichtung nach einem der Ansprüche 1 bis 4, dadurch gekennzeichnet, daß an die verformbaren Bereiche (6,7,14,15,16) bzw. an die Meßleitung (2) unter Zwischenschaltung einer Ventilanordnung (12), insbesondere eines Rückschlagventils, eine Druckquelle (13) anschließbar ist.

6. Einrichtung nach einem der Ansprüche 1 bis 5, dadurch gekennzeichnet, daß als Fluid physiologisch unbedenkliche Flüssigkeiten, insbesondere aqua bidestillata, eingesetzt sind.

7. Einrichtung nach einem der Ansprüche 1 bis 6, dadurch gekennzeichnet, daß in radialer Richtung voneinander getrennte Kammern (14, 15, 16) angeordnet sind, welche mit unterschiedlichem Druck mit Fluid beaufschlagbar sind.

8. Einrichtung nach einem der Ansprüche 1 bis 7, dadurch gekennzeichnet, daß der Sondenkopf (1) wenigstens über einen Teil seiner Länge von einem Hohlzylinder (17) gebildet ist, welcher einen in Längsrichtung verlaufenden Spalt (18) aufweist, und daß am Innenumfang des Zylinders wenigstens ein mit der Meßleitung (2) verbundener Dehnungsmeßstreifen (19) angeordnet ist.

9. Einrichtung nach Anspruch 8, dadurch gekennzeichnet, daß der Dehnungsmeßstreifen (19) dem Spalt (18) gegenüberliegend angeordnet ist.

10. Einrichtung nach einem der Ansprüche 1 bis 7, dadurch gekennzeichnet, daß der Sondenkopf (1) wenigstens über einen Teil seiner Länge von einem Hohlzylinder (17) gebildet ist, welcher einen in Längsrichtung verlaufenden Spalt (18) aufweist, und daß im Bereich des Spaltes mit der Meßleitung (2) verbundene, elektrisch leitfähige Platten (21) angeordnet sind.

11. Einrichtung nach einem der Ansprüche 1 bis 10, dadurch gekennzeichnet, daß der Sondenkopf (1) mit einem elastischen Material (20), beispielsweise Schaumstoff, Kautschuk oder dgl., ummantelt ist.

12. Einrichtung nach einem der Ansprüche 1 bis 11, dadurch gekennzeichnet, daß der Sondenkopf (1) elastische Bereiche (23) aufweist, in welchen elektrisch leitfähiges Material, beispielsweise Kohlenstoffstaub, elektrisch leitende Flüssigkeiten oder Lösungen oder dgl., enthalten ist, und daß in dem elektrisch leitfähigen Material (23) mit der Meßleitung (2) verbundene Elektroden (24) eingebettet sind.

13. Einrichtung nach einem der Ansprüche 1 bis 12, dadurch gekennzeichnet, daß an dem dem freien Ende des Sondenkopfes (1) gegenüberliegenden Ende ein Positionieranschlag (25) am Sondenkopf angeordnet ist.

14. Einrichtung nach Anspruch 13, dadurch gekennzeichnet, daß der Positionieranschlag (25) von einer insbesondere flexiblen Kunststoffplatte gebildet ist, deren Außendurchmesser den Durchmesser des Sondenkopfes (1) um wenigstens ein Drittel dieses Durchmessers übersteigt.

15. Einrichtung nach Anspruch 13 oder 14, dadurch gekennzeichnet, daß am Positionieranschlag (25) ein Griff (26) vorgesehen ist.

16. Einrichtung nach einem der Ansprüche 13 bis 15, dadurch gekennzeichnet, daß der Positionieranschlag (25) in variablem Abstand vom freien Ende des Sondenkopfes (1) an diesem festlegbar ist.

## Claims

1. Device for monitoring the contractibility of the pelvic floor muscles in which a probing head (1) can be connected to a display device (3) by way of at least one measurement line (2), and the probing head (1) is formed as an extended body that incorporates a constriction in its middle area (4) and wherein the probing head (1) has on its periphery at least one pressure sensor (19, 21, 24) or areas (6, 7, 14, 15, 16) that are separated from each other and can be deformed elastically against a fluid, characterized in that the middle constricted area (4) of the probing head (1) has an elastically deformable covering, the deformability of which is greater than the deformability of the areas (7, 8) of the probing head that are adjacent to the middle area (4).

2. Device according to claim 1, characterized in that the measurement line (2) is formed as a fluid line and can be connected to a fluid gauge chamber (3) and to a display device that is connected with the fluid gauge chamber.

3. Device according to claim 1 or 2, characterized in that the middle area (4) incorporates stiffenings (10) that extend in the axial direction, in particular a rod that is connected to the less-deformable areas (7, 8) that are adjacent to the middle area and passes through the constricted area in an axial direction.

4. Device according to any of the claims 1 to 3, characterized in that the probing head (1) has at its free end at least one separate pressure sensor (9) or a chamber that is defined by an elastically deformable membrane, wherein a separate measurement line can be connected to a measuring and display device.

5. Device according to any of the claims 1 to 4, characterized in that a pressure source (13) can be connected to the deformable areas (6, 7, 14, 15, 16) or to the measurement line (2) incorporating a valve arrangement (12), in particular a nonreturn valve.

6. Device according to any of the claims 1 to 5, characterized in that physiologically safe liquids, in particular aqua bidestillata, are used as the fluid.

7. Device according to any of the claims 1 to 6, characterized in that chambers (14, 15, 16) are so arranged as to be separated from each other in the radial direction, these being acted upon with fluid at a different pressure.

8. Device according to any of the claims 1 to 7, characterized in that the probing head (1) is formed at least to part of its length by a hollow cylinder (17) that has a gap (18) that extends in a longitudinal direction and in that at least one wire strain gauge (19) that is connected with the measurement line (2) is arranged on the inside periphery of the cylinder.

9. Device according to claim 8, characterized in that the wire strain gauge (19) is arranged opposite the gap (18).

10. Device according to any of the claims 1 to 7, characterized in that the probing head (1) is formed to at least part of its length by a hollow cylinder (17) that incorporates a gap (18) that extends in the longitudinal direction and in that in the area of the gap there are arranged electrically conductive plates (21) that are connected to the measurement line (2).

11. Device according to any of the clalms 1 to 10, characterized in that the probing head (1) is covered with an elastic material (20), for example, foam, rubber, or the like.

12. Device according to any of the claims 1 to 11, characterized in that the probing head (1) incorporates elastic areas (23) that contain electrically conductive material, for example carbon dust, electrically conductive liquids or solutions, or the like, and in that electrodes (24) that are connected with the measurement line (2) are embedded in the electrically conductive material (23).

13. Device according to any of the claims 1 to 12, characterized in that a positioning stop (25) is arranged on the probing head at the end that is opposite to the free end of said probing head (1).

14. Device according to claim 13, characterized in that the positioning stop (25) is formed from a particularly flexible plastic plate, the outside diameter of which exceeds the diameter of the probing head (1) by at least one-third of this diameter.

15. Device according to claim 13 or 14, characterized in that there is provided a grip (26) on the positioning stop (25).

16. Device according to any of the claims 13 to 15, characterized in that the positioning stop (25) can be secured on the probing head (1) at varying distances from the free end of said probing head (1).

## Revendications

1. Dispositif de contrôle de la contractilité de la musculature du plancher pelvien, dans lequel une tête sonde (1) peut être reliée à un appareil indicateur (3) par au moins une ligne de mesure (2), cette tête sonde (1) est un corps allongé qui présente un étranglement dans sa zone médiane (4), et cette tête sonde (1) présente sur son pourtour au moins un capteur de pression (19, 21, 24) ou des zones (6, 7, 14, 15, 16) séparées les unes des autres et déformables élastiquement vis-à-vis d'un fluide, caractérisé par le fait que la zone médiane étranglée (4) de la tête sonde (1) présente une enveloppe déformable élastiquement dont la déformabilité est supérieure à celle des zones partielles (7, 8) de la tête sonde contiguës à la zone médiane (4).

2. Dispositif selon la revendication 1, caractérisé par le fait que la ligne de mesure (2) est une conduite de fluide et peut être reliée à une capsule manométrique (3) et à un appareil indicateur relié à celle-ci.

3. Dispositif selon l'une des revendications 1 et 2, caractérisé par le fait que la zone médiane (4) présente des renforts (10) s'étendant dans la direction axiale de la tête sonde, en particulier une tige traversant axialement la zone étranglée et jointe aux zones moins déformables (7, 8) voisines de la zone médiane.

4. Dispositif selon l'une des revendications 1 à 3, caractérisé par le fait que la tête sonde (1) présente à son extrémité libre au moins un capteur de pression séparé (9) ou une chambre limitée par une membrane déformable élastiquement, une ligne de mesure séparée pouvant être reliée à un appareil de mesure et indicateur.

5. Dispositif selon l'une des revendications 1 à 4, caractérisé par le fait qu'une source de pression (13) peut être reliée aux zones déformables (6, 7, 14, 15, 16) ou à la ligne de mesure (2) avec intercalation d'une vanne (12), en particulier d'un clapet de non-retour.

6. Dispositif selon l'une des revendications 1 à 5, caractérisé par le fait que comme fluide sont utilisés des liquides physiologiquement sans danger, en particulier de l'eau bidistillée.

7. Dispositif selon l'une des revendications 1 à 6, caractérisé par le fait qu'il est prévu des chambres séparées les unes des autres dans la direction radiale (14, 15, 16) qui peuvent être alimentées en fluide à des pressions différentes.

8. Dispositif selon l'une des revendications 1 à 7, caractérisé par le fait que la tête sonde (1) est formée au moins sur une partie de sa longueur d'un cylindre creux (17) qui présente une fente longitudinale (18), et que sur le pourtour intérieur de ce cylindre est placée au moins une jauge extensométrique (19) jointe à la ligne de mesure (2).

9. Dispositif selon la revendication 8, caractérisé par le fait que la jauge extensométrique (19) est opposée à la fente (18).

10. Dispositif selon l'une des revendications 1 à 7, caractérisé par le fait que la tête sonde (1) est formée au moins sur une partie de sa longueur d'un cylindre creux (17) qui présente une fente longitudinale (18), et que dans la zone de cette fente sont placées des plaques conduisant l'électricité (21) reliées à la ligne de mesure (2).

11. Dispositif selon l'une des revendications 1 à 10, caractérisé par le fait que la tête sonde (1) est enveloppée d'une matière élastique (20), par exemple de matière alvéolaire, de caoutchouc ou d'une matière semblable.

12. Dispositif selon l'une des revendications 1 à 11, caractérisé par le fait que la tête sonde (1) présente des zones élastiques (23) qui contiennent une matière conduisant l'électricité, par exemple de la poudre de carbone ou des liquides ou des solutions conduisant l'électricité, et que dans cette matière conduisant l'électricité (23) sont noyées des électrodes (24) reliées à la ligne de mesure (2).

13. Dispositif selon l'une des revendications 1 à 12, caractérisé par le fait qu'à l'extrémité opposée à l'extrémité libre de la tête sonde (1) est placée sur la tête sonde une butée de positionnement (25).

14. Dispositif selon la revendication 13, caractérisé par le fait que la butée de positionnement (25) est formée d'une plaque en plastique en particulier flexible dont le diamètre extérieur dépasse le diamètre de la tête sonde (1) d'au moins un tiers de ce diamètre.

15. Dispositif selon l'une des revendications 13 et 14, caractérisé par le fait que sur la butée de positionnement (25) est prévue une poignée (26).

16. Dispositif selon l'une des revendications 13 à 15, caractérisé par le fait que la butée de positionnement (25) peut être fixée à la tête sonde (1) à une distance variable de l'extrémité libre de celle-ci.
